# EUROPEAN PATENT APPLICATION

(11) **EP 4 796 119 A1**
(43) Date of publication of application: **26.08.2026**
(21) Application number: 24879699.7
(22) Date of filing: 15.10.2024
(51) Int. Cl.: A61K 35/76, A61K 39/00, A61K 39/385, A61P 3/04

(54) **TREATMENT OF OBESITY**

(30) Priority: 16.10.2023 US 202363590604 P
(71) Applicant: Cyn-K Bio, Inc., Kyoto-shi, Kyoto 606-8303 (JP); Kyoto University, Kyoto-shi, Kyoto 606-8501 (JP)
(72) Inventor: GOTO, Kazuya, Kyoto-shi, Kyoto 606-8501 (JP); UENO, Ryuji, Easton, Maryland 21601 (US); AKAHATA, Wataru, Kensington, Maryland 20895 (US)
(74) Representative: Kraus & Lederer PartGmbB
(86) International application number: PCT/JP2024/036667
(87) International publication number: WO 2025/084276

(57) **Abstract**

The present disclosure provides a method for treating or preventing obesity in a mammalian subject in need thereof, which comprises an effective amount of a virus-like particle comprising a viral structural protein and a galectin-3 antigen to the subject.

## Description

### TECHNICAL FIELD

The present disclosure relates to a method for the treatment of obesity in a mammalian subject in need thereof. The present disclosure also relates to a method for reducing or suppressing the increase of visceral fat in a mammalian subject.

### BACKGROUND ART

Overweight and obesity are defined as abnormal or excessive fat accumulation that presents a risk to health. A body mass index (BMI) over 25 is considered overweight, and over 30 is obese. The issue has grown to epidemic proportions, with over 4 million people dying each year as a result of being overweight or obese in 2017 according to the global burden of disease.

Rates of overweight and obesity continue to grow in adults and children. From 1975 to 2016, the prevalence of overweight or obese children and adolescents aged 5-19 years increased more than four-fold from 4% to 18% globally.

Obesity is one side of the double burden of malnutrition, and today more people are obese than underweight in every region except sub-Saharan Africa and Asia. Once considered a problem only in high-income countries, overweight and obesity are now dramatically on the rise in low- and middle-income countries, particularly in urban settings. The vast majority of overweight or obese children live in developing countries, where the rate of increase has been more than 30% higher than that of developed countries

Galectins are members of the lectin family, which show high affinity for β-galactosides. There have been about 15 galectins discovered in mammals, encoded by the LGALS genes, which are numbered in a consecutive manner. Currently only galectin-1, -2, -3, - 4, -7, -7B, -8, -9, -9B, 9C, -10, -12, -13, -14, and -16 have been identified in humans.

Galectin-3 (Gal-3) is a member of a highly conserved family of animal lectins binding to β-galactoside-containing glycoconjugates (glycoprotein or glycolipids) (Non Patent Literature 1: Henderson NC 2006, Non Patent Literature 2: Mourad-Zeidan AA 2008). Gal-3 is unique among other Galectin-family proteins in its structure composed of two domains: a carboxyl-terminal domain that contains the carbohydrate-binding region and an amino-terminal domain consisting primarily of tandem repeat of nine amino acids to cross-link carbohydrate and noncarbohydrate ligands (Non Patent Literature 3: Barondes SH 1994). It is secreted by various types of cells including monocytes, macrophages and epithelial cells, but mainly by macrophage (Non Patent Literature 4: Reynolds 2005, Non Patent Literature 5: MacKinnon AC 2008). Secreted extracellular Gal-3 forms homodimer or pentamer and it is important for the biological function. The N-terminal domain of galectin-3 has been demonstrated to be important for the formation of protein oligomers (Non Patent Literature 6: Dumic J 2006, Non Patent Literature 7: Kuklinski S 1998, Non Patent Literature 8: Lepur A 2012). The released protein can function as an extracellular molecule to activate cells, mediate cell-cell and cell-ECM interactions, induce migration of various types of cell, and negatively regulate T cell receptor signaling (Non Patent Literature 9: Yang RY 1996). Gal-3 was shown to be increased in various models of fibrotic diseases and patients including lung fibrosis, liver fibrosis, systemic sclerosis and cardiac fibrosis (Non Patent Literature 10: Henderson NC 2008, Non Patent Literature 11: Barman, et al. 2019, Non Patent Literature 12: Nishi Y 2007, Non Patent Literature 13: De Boer 2010). This suggests that Gal-3 may be an important mediator of and effective therapeutic target for tissue fibrosis. To date, preclinical and clinical studies of investigational Galectin-3 inhibitors showed protection against fibrotic disorders (Non Patent Literature 14: Nikhil Hirani 2017, Non Patent Literature 15: Yu L 2013).

One of the major success stories in medicine during the last 100-150 years is vaccines targeting various infectious diseases. Vaccines have, together with antibiotics, likely been more important for human health than any other factor affecting human health. Because of the success of vaccines, the interest in using vaccine technology for the treatment of noninfectious diseases such as allergies, autoimmunity, and cancer is increasing. However, the targets for these diseases are in general self-antigens, which may pose problems with efficacy. It is considerably difficult to induce a strong antibody response to a self-antigen compared with a non-self-antigen-bacterial, viral, or parasite protein because of tolerance mechanisms (Non Patent Literature 16: Hellman 2008, Non Patent Literature 17: Falk Saupe 2015).

Alphaviruses comprise a set of genetically, structurally, and serologically related mosquito-borne viruses of the Togaviridae family. The alphaviruses include Eastern Equine Encephalitis Virus (EEEV), Venezuelan Equine Encephalitis Virus (VEEV), Everglades Virus, Mucambo Virus, Pixuna Virus, Western Equine Encephalitis Virus (WEEV), Sindbis Virus, Semliki Forest Virus, Middleburg Virus, Chikungunya Virus (CHIKV), O'nyong-nyong Virus, Ross River Virus, Barmah Forest Virus, Getah Virus, Sagiyama Virus, Bebaru Virus, Mayaro Virus, Una Virus, Aura Virus, Whataroa Virus, Babanki Virus, Kyzylagach Virus, Highlands J virus, Fort Morgan Virus, Ndumu Virus, and Buggy Creek Virus. Structural subunits containing a single viral protein, capsid, associate with the RNA genome in an icosahedral nucleocapsid. In the virion, the capsid is surrounded by a lipid envelope covered with a regular array of transmembrane protein spikes, each of which consists of a heterodimeric complex of two glycoproteins, E1 and E2.

Virus-like particles (VLPs) are multiprotein structures that mimic the organization and conformation of authentic native viruses but lack the viral genome, potentially yielding safer and cheaper vaccine candidates. A handful of prophylactic VLP-based vaccines is currently commercialized worldwide: GlaxoSmithKline's EngerixR (hepatitis B virus) and CervarixR (human papillomavirus), and Merck and Co., Inc.'s Recombivax HBR (hepatitis B virus) and GardasilR (human papillomavirus) are some examples. Other VLP-based vaccine candidates are in clinical trials or undergoing preclinical evaluation, such as, influenza virus, parvovirus, Norwalk and various chimeric VLPs. Many others are still restricted to small-scale fundamental research, despite their success in preclinical tests. The implications of large-scale VLP production are discussed in the context of process control, monitoring and optimization. The main up- and down-stream technical challenges are identified and discussed accordingly. Successful VLP-based vaccine blockbusters are briefly presented concomitantly with the latest results from clinical trials and the recent developments in chimeric VLP-based technology for either therapeutic or prophylactic vaccination.

Up to now, VLP-based vaccines have been produced for more than 30 different viruses that infect human and other animals. The examples include AAV (Adeno-associated virus), H5N3 (Avian influenza), BFDV (Budgerigar fledgling disease virus), BTV (Bluetongue virus), Ebola, Enterovirus 71, GHPV (Goose hemorrhagic polyoma virus), HBV (Hepatitis B virus), HCV (Hepatitis C virus), HDV (Hepatitis δ virus), HEV (Hepatitis E virus), HIV, HPV (Human papillomavirus), IBDV (Infectious bursal disease virus), Influenza A, Influenza A H1N1, Influenza A H3N2, JC polymavirus, Marburg, MS2, IPCV (Indian peanut clump virus), NDV (Newcastle disease virus), No (Norovirus) Nv (Norwalk virus), PhMV (Physalis mottle virus), Polymavirus, PPV (Porcine parvovirus), RHDV (Rabbit hemorrhagic disease virus), Rotavirus, SARS, SIV (Simian immunodeficiency virus), SV40 (Simian virus 40), SVDV (Swine vesicular disease virus) and so on. (Non Patent Literature 18: Expert Rev. Vaccines 9(10), 1149-1176, 2010).

US patent No. 9,353,353 discloses a virus-like particle (VLP) comprising one or more Chikungunya viral structural proteins which is useful for formulating a vaccine or antigenic composition for Chikungunya that induces immunity to an infection or at least one symptom thereof. US patent No. 9,487,563 discloses modified alphavirus or flavivirus virus-like particles (VLPs) and methods for enhancing production of modified VLPs for use in the prevention or treatment of alphavirus and flavivirus-mediated diseases. US patent No. 9,249,191 discloses a Chikungunya virus (CHIKV) or Venezuelan equine encephalitis virus (VEEV) virus-like particle, wherein said virus-like particle contains at least one antigen inserted into an E2 envelope protein to form a fusion protein. US patent No. 9,969,986 discloses an alphavirus virus-like particle, wherein said virus-like particle comprises an alphavirus structural protein that comprises an envelope protein E3, wherein said envelope protein E3 is modified to contain at least one foreign antigen inserted into furin cleavage site thereof (these cited references are herein incorporated by reference).

### [CITATION LIST]

### [Patent Literature]

[PTL 1] US patent No. 9,353,353
[PTL 2] US patent No. 9,487,563
[PTL 3] US patent No. 9,249,191
[PTL 4] US patent No. 9,969,986
[PTL 5] US patent publication No. 2005/0118191
[PTL 6] WO 2022/225057

### [Non Patent Literature]

[NPL 1] Henderson NC, Mackinnon AC, Farnworth SL, Poirier F, Russo FP, Iredale JP, Haslett C, Simpson KJ, Sethi T. 2006. "Galectin-3 regulates myofibroblast activation and hepatic fibrosis." Proc Natl Acad Sci U S A 103(13):5060-5.
[NPL 2] Mourad-Zeidan AA, Melnikova VO, Wang H, Raz A, and Bar-Eli M. 2008. "Expression profiling of Galectin-3-depleted melanoma cells reveals its major role in melanoma cell plasticity and vasculogenic mimicry." Am J Pathol 173:1839-1852.
[NPL 3] Barondes SH, Cooper DN, Gitt MA, Leffler H. 1994. "Galectins. Structure and function of a large family of animal lectins." J Biol Chem 269:20807-20810.
[NPL 4] Herbert Y. Reynolds, "Lung Inflammation and Fibrosis, An Alveolar Macrophage-centered Perspective from the 1970s to 1980s" Am J Respir Crit Care Med Vol 171. pp 98-102, 2005
[NPL 5] MacKinnon AC, Farnworth SL, Hodkinson PS, Henderson NC, Atkinson KM, Leffler H, Nilsson UJ, Haslett C, Forbes SJ, and Sethi T. 2008. "Regulation of alternative macrophage activation by galectin-3." J Immunol 180:2650-2658.
[NPL 6] Dumic J, Dabelic S, Flougel M. 2006. "Galectin-3: An open-ended story." BBA-Gen Subjects 1760:616-635.
[NPL 7] Kuklinski S, Probstmeier R. 1998. "Homophilic binding properties of galectin-3: Involvement of the carbohydrate recognition domain." J Neurochem 70:814-823.
[NPL 8] Lepur A, Salomonsson E, Nilsson UJ, Leffler H. 2012. "Ligand induced galectin-3 protein self-association." J Biol Chem 287:21751-21756.
[NPL 9] Yang RY, Hsu DK, and Liu FT. 1996. "Expression of galectin-3 modulates T-cell growth and apoptosis." Proc Natl Acad Sci USA 93:6737-6742.
[NPL 10] Henderson NC, Mackinnon AC, Farnworth SL, Kipari T, Haslett C, Iredale JP, Liu FT, Hughes J, Sethi T. 2008. "Galectin-3 expression and secretion links macrophages to the promotion of renal fibrosis." Am J Pathol. 172(2):288-98.
[NPL 11] Barman, Scott A, Xueyi Li, Stephen Haigh, Dmitry Kondrikov, Keyvan Mahboubi, Zsuzsanna Bordan, and Stepp W David. 2019. "Galectin-3 is Expressed in Vascular Smooth Muscle Cells and Promotes Pulmonary Hypertension through changes in Proliferation, Apoptosis and Fibrosis." Epub ahead of print.
[NPL 12] Nishi Y, Sano H, Kawashima T, Okada T, Kuroda T, Kikkawa K, Kawashima S, Tanabe M, Goto T, Matsuzawa Y, Matsumura R, Tomioka H, Liu FT, Shirai K. 2007. "Role of Galectin-3 in Human Pulmonary Fibrosis" Allergol Int. 56(1):57-65.
[NPL 13] De Boer, R. A., Yu, L., and van Veldhuisen, D. J. 2010. "Galectin-3 in cardiac remodeling and heart failure." Curr. Heart Fail. Rep. 7, 1-8.
[NPL 14] Nikhil Hirani, Alison Mackinnon , Lisa Nicol , Jeremy Walker , Paul Ford , Hans Schambye , Anders Pederson, Ulf Nilsson, Hakon Leffler , Tracy Thomas , Danielle Francombe , John Simpson , Michael Gibbons , Toby M. Maher. 2017. "TD139, A Novel Inhaled Galectin-3 Inhibitor for the Treatment of Idiopathic Pulmonary Fibrosis (IPF). Results from the First in (IPF) Patients Study." American Journal of Respiratory and Critical Care Medicine 195:A7560.
[NPL 15] Yu L, Ruifrok WP, Meissner M, Bos EM, van Goor H, Sanjabi B, van der Harst P, Pitt B, Goldstein IJ, Koerts JA, et al. 2013. "Genetic and pharmacological inhibition of galectin-3 prevents cardiac remodeling by interfering with myocardial fibrogenesis." Circ Heart Fail 6:107-117.
[NPL 16] Hellman, L. 2008. "Therapeutic vaccines against IgE-mediated allergies." Expert Rev Vaccines 7(2):193-208.
[NPL 17] Falk Saupe, Elisabeth J. M. Huijbers, Tobias Hein, Julia Femel, Jessica Cedervall, Anna-Karin Olsson, and Lars Hellman. 2015. "Vaccines targeting self-antigens: mechanisms and efficacy-determining parameters." FASEB J 29(8):3253-62.
[NPL 18] Antonio Roldao, Maria Candida M, Mellado, Leda R, Castilho, Manuel JT Carrondo and Paula M Alves "Virus-like particles in vaccine development" Expert Rev. Vaccines 9(10), 1149-1176, 2010
[NPL 19] Goldfarb, D., and N. Michaud (1991) "Pathways for the nuclear transport of proteins and RNAs" Trends Cell Biol. 1, 20-24
[NPL 20] Gorlich, D., and I. W. Mattaj (1996) "Nucleocytoplasmic Transport" Science 271, 1513-1518
[NPL 21] Schneider, J. et al. (1988) "A Mutant SV40 Large T Antigen Interferes with Nuclear Localization of a Heterologous Protein" Cell 54,117-125
[NPL 22] Dingwall et al. (1988) "The Nucleoplasmin Nuclear Location Sequence Is Larger and More Complex than That of SV-40 Large T Antigen" Cell Biol. 107 (3): 841-9

### SUMMARY OF INVENTION

An object of the present disclosure is to provide a method for the treatment or prevention of obesity in a mammalian subject in need thereof. Another object of the present disclosure is to provide a method for reducing or suppressing the increase of visceral fat in a mammalian subject in need thereof. Still another object is to provide a pharmaceutical composition or use for the treatment or prevention of obesity.

Namely, the present disclosure provides a method for the treatment or prevention of obesity in a mammalian subject in need thereof, which comprises administering to the subject an effective amount of a virus-like particle comprising a viral structural protein and a galectin-3 antigen. The disclosure also provides a method for reducing or preventing the increase of visceral fat in a subject in need thereof, which comprises administering to the subject an effective amount of a virus-like particle comprising a viral structural protein and a galectin-3 antigen.

The present disclosure also provides a pharmaceutical composition for the treatment or prevention of obesity, which comprises a virus-like particle comprising a viral structural protein and a galectin-3 antigen.

The present disclosure also provides use of a virus-like particle comprising a viral structural protein and a galectin-3 antigen for the manufacture of a medicament for the treatment or prevention of obesity. .

### BRIEF DESCRIPTION OF DRAWINGS

[Fig. 1]
   Anti-mouse galectin-3 (Gal-3) antibody in the serum of VEEV-Gal-3-VLP immunized mice
[Fig. 2]
   Effects of VEEV-Gal-3-VLP on body weight in C57BL/6J mice.
[Fig. 3]
   Effects of VEEV-Gal-3-VLP on visceral fat in C57BL/6J mice.
[Fig. 4]
   Anti-human Galectin-3 (Gal-3) antibody in the serum of VEEV-Gal-3-VLP immunized mice

### DETAILED DESCRIPTION

As used herein, the term "galectin-3 antigen" refers to any antigenic structure derived from galectin-3 protein which can be recognized by the immune system and/or that stimulates a cell-mediated immune response and/or stimulates the generation of antibodies specific to the antigen. The galectin-3 epitope peptide may be a fragment of a naturally occurring galectin-3 protein, or a fragment of a naturally occurring galectin protein with some modifications. The naturally occurring galectin-3 protein may preferably be human galectin protein and especially, human galectin-3 protein. In one embodiment, the modified fragment has at least 80%, 85%, 90%, 95% or 98% amino acid sequence identity to a fragment of the naturally occurring galectin-3 protein. In one embodiment, the modified peptide fragment is a mutant where at most 10% of the amino acids are deleted, substituted, and/or added based on a fragment of the naturally occurring galectin-3 protein.

### Galectin-3 [Homo sapiens] (GenBank Accession No: AAB86584.1)





 (SEQ ID NO: 1)

Examples of galectin-3 antigens, i.e. galectin-3 epitope peptides may include the followings:

ADNFSLHDALSGSGNPNPQGWPGAWGNQPA (SEQ ID NO: 2)
YPGASYPGAYPGQAPPGAYPGQAPPGAYPGAPGA (SEQ ID NO: 3)
YPGASYPGAYPGQAPPGAYPGQAPPGAYPGAPGAYPGAPAPGVYPGPPSGPGAY
PSSGQPSATGAYPATGPYGA (SEQ ID NO: 4)
ADSFSLNDALAGSGNPNPQGYPGAWGNQPA (SEQ ID NO: 5)

Gal-3 epitope peptide SEQ ID NO: 2 consists of human N-term Gal-3 epitope peptide. Gal-3 epitope peptides SEQ ID NOs: 3 and 4 consisting of human Gal-3 N-term repeat sequence epitope peptide and SEQ ID NO: 4 has longer repeat sequence than that of SEQ ID NO: 3. SEQ ID NO: 5 encodes mouse Gal-3 N-term peptide

In WO 2022/225057 (the contents of this document is herein incorporated by reference), VLPs bearing the above Gal-3 epitope peptides were prepared and immunogenicity of those VLPs were confirmed.

### Alphavirus

As used herein "alphavirus" is meant to refer to RNA-containing viruses that belong to the Togaviridae family of viruses. Exemplary Togaviridae viruses include but are not limited to Eastern Equine Encephalitis Virus (EEEV), Venezuelan Equine Encephalitis Virus (VEEV), Everglades Virus, Mucambo Virus, Pixuna Virus, Western Equine Encephalitis Virus (WEEV), Sindbis Virus, Semliki Forest Virus, Middleburg Virus, Chikungunya Virus (CHIKV), O'nyong-nyong Virus, Ross River Virus, Barmah Forest Virus, Getah Virus, Sagiyama Virus, Bebaru Virus, Mayaro Virus, Una Virus, Aura Virus, Whataroa Virus, Babanki Virus, Kyzylagach Virus, Highlands J virus, Fort Morgan Virus, Ndumu Virus, Buggy Creek Virus, Ockelbo virus.

By "viral structural protein" is meant a polypeptide or fragment thereof having at least about 80% amino acid sequence identity to a naturally occurring viral capsid or envelope protein and having immunogenic activity in a mammal. In one embodiment, the alphavirus structural protein has at least about 85%, 90%, 95% or greater amino acid sequence identity with the above discussed alphaviruses.

A viral structural protein and a galectin-3 antigen may be directly or indirectly fused. In one embodiment, one or two linkers may intervene between N-terminal residue of an antigen and a viral structural protein and/or between C-terminal residue of an antigen and a viral structural protein.

An antigen or a viral structural protein can be truncated and replaced by short linkers. In some embodiments, an antigen or a viral structural protein include one or more peptide linkers. Typically, a linker consists of from 2 to 25 amino acids (e.g. 2, 3, 4, 5 or 6 amino acids). Usually, it is from 2 to 15 amino acids in length, although in certain circumstances, it can be only one, such as a single glycine residue.

In one embodiment, a nucleic acid molecule, in which polynucleotide encoding the viral structural protein is genetically fused with polynucleotide encoding the antigen, is expressed in a host cell (e.g. mammalian cells (e.g. 293F cells)) so that the first attachment site and the second attachment site are linked through a peptide bond. In this case, the viral structural protein and the antigen are linked through a peptide bond. Relating to this embodiment, the first attachment site and/or the second attachment site may be genetically modified from the original protein or antigen. For example, the first attachment site is modified from the viral structural protein so that through a linker peptide including SG, GS, SGG, GGS and SGSG, the protein is conjugated with the antigen. When the viral structural protein is chemically conjugated with the antigen, the first attachment site and the second attachment site may be linked through a chemical cross-linker which is a chemical compound. Examples of the cross-linker include, but are not limited to, SMPH, Sulfo-MBS, Sulfo-EMCS, Sulfo-GMBS, Sulfo-SIAB, Sulfo-SMPB, Sulfo-SMCC, SVSB, SIA and other cross-linkers available from the Pierce Chemical Company.

Preferably, an antigen may be linked to the Chikungunya viral structural protein or Venezuelan equine encephalitis viral structural protein as a fusion protein produced by way of genetic engineering.

A Chikungunya viral structural protein or Venezuelan equine encephalitis viral structural protein used in the present application may be a Chikungunya or Venezuelan equine encephalitis virus envelope protein or a capsid or a complex of one or more envelope proteins and/or a capsid protein.

Examples of Chikungunya virus include, but are not limited to, strains 37997 and strain LR2006 OPY-1.

Examples of Venezuelan equine encephalitis virus include, but are not limited to, strain TC-83.

Chikungunya viral structural protein or Venezuelan equine encephalitis viral structural protein used in the present application may be a naturally occurring viral structural protein or modified protein thereof. The modified protein may be a fragment of the naturally occurring viral structural protein. In one embodiment, the modified protein has at least 70%, 75%, 80%, 85%, 90%, 95% or 98% amino acid sequence identity to a naturally occurring viral capsid and/or envelope protein. In one embodiment, the modified protein is a mutant where at most 10% of the amino acids are deleted, substituted, and/or added based on a naturally occurring viral capsid and/or envelope protein. For example, K64A or K64N mutation may be introduced into a capsid of Venezuelan equine encephalitis viral structural protein used in the present application.

Chikungunya or Venezuelan equine encephalitis viral structural protein may consist of or comprise a capsid, E3, E2 and E1 proteins. E3 and E2 proteins may be expressed together so that E2 and E3 can form one protein.

Examples of Chikungunya viral structural protein include, but are not limited to, Capsid-E3-E2-E1 of Chikungunya virus Strain 37997, and Capsid-E3-E2-E1 of Chikungunya virus LR2006 OPY-1.

Examples of Venezuelan equine encephalitis viral structural protein include, but are not limited to, Capsid-E3-E2-E1 of Venezuelan equine encephalitis virus Strain TC-83.

An exemplary Chikungunya viral structural protein sequence is provided at Genbank Accession No. ABX40006.1, which is described below (SEQ ID NO: 6):

[Chem. 1]
mefiptqtfynrryqprpwtprptiqvirprprpqrqagqlaqlisavnkltmravpqqkpr
rnrknkkqkqkqqapqnntnqkkqppkkkpaqkkkkpgrrermcmkiendcifevkhegkvt
gyaclvgdkvmkpahvkgtidnadlaklafkrsskydlecaqipvhmksdaskfthekpegy
ynwhhgavqysggrftiptgagkpgdsgrpifdnkgrvvaivlgganegartalsvvtwnkd
ivtkitpegaeewslaipvmcllanttfpcsqppctpccyekepeetlrmlednvmrpgyyq
llqasltcsphrqrrstkdnfnvykatrpylahcpdcgeghschspvalerirneatdgtlk
iqvslqigiktddshdwtklrymdnhmpadaeraglfvrtsapctitgtmghfilarcpkge
tltvgftdsrkishscthpfhhdppvigrekfhsrpqhgkelpcstyvqstaatteeievhm
ppdtpdrtlmsqqsgnvkitvngqtvrykcncggsnegltttdkvinnckvdqchaavtnhk
kwqynsplvprnaelgdrkgkihipfplanvtcrvpkarnptvtygknqvimllypdhptll
syrnmgeepnyqeewvmhkkevvltvptegleevtwgnnepykywpqlstngtahghpheiil
yyyelyptmtvvvvsvatfillsmvgmaagmcmcarrrcitpyeltpgatvpfllsliccir
takaatyqeaaiylwneqqplfwlqaliplaalivlcnclrllpcccktlaflavmsvgaht
vsayehvtvipntvgvpyktlvnrpgyspmvlemellsvtleptlsldyitceyktvipspy
vkccgtaeckdknlpdysckvftgvypfmwggaycfcdaentqlseahveksesckteafasa
yrahtasasaklrvlyqgnnitvtayangdhavtvkdakfivgpmssawtpfdnkivvykgd
vynmdyppfgagrpgqfgdiqsrtpeskdvyantqlvlqrpavgtvhvpysqapsgfkywlk
ergaslqhtapfgcqiatnpvravncavgnmpisidipeaaftrvvdapsltdmscevpact
hssdfggvaiikyaaskkgkcavhsmtnavtireaeievegnsqlqisfstalasaefrvqv
cstqvhcaaechppkdhivnypashttlgvqdisatamswvqkitggvglvvavaalilivv
lcvsfsrh

Another exemplary Chikungunya viral structural protein sequence is provided at Genbank Accession No. ABX40011.1, which is described below (SEQ ID NO: 7):

[Chem. 2]
mefiptqtfynrryqprpwaprptiqvirprprpqrqagqlaqlisavnkltmravpqqkpr
rnrknkkqrqkkqapqndpkqkkqppqkkpaqkkkkpgrrermcmkiendcifevkhegkvm
gyaclvgdkvmkpahvkgtidnadlaklafkrsskydlecaqipvhmksdaskfthekpegy
ynwhhgavqysggrftiptgagkpgdsgrpifdnkgrvvaivlgganegartalsvvtwnkd
ivtkitpegaeewslalpvlcllanttfpcsqppctpccyekepestlrmlednvmrpgyyq
llkasltcsphrqrrstkdnfnvykatrpylahcpdcgeghschspialerirneatdgtlk
iqvslqigiktddshdwtklrymdshtpadaeragllvrtsapctitgtmghfilarcpkge
tltvgftdsrkishtcthpfhheppvigrerfhsrpqhgkelpcstyvqstaataeeievhm
ppdtpdrtlmtqqsgnvkitvngqtvrykcncggsnegltttdkvinnckidqchaavtnhk
nwqynsplvprnaelgdrkgkihipfplanvtcrvpkarnptvtygknqvtmllypdhptll
syrnmgqepnyheewvthkkevtltvptegleevtwgnnepykywpqmstngtahghpheiil
yyyelyptmtvvivsvasfvllsmvgtavgmcvcarrrcitpyeltpgatvpfllsllccvr
ttkaatyyeaaaylwneqqplfwlqaliplaalivlcnclkllpcccktlaflavmsigaht
vsayehvtvipntvgvpyktlvnrpgyspmvlemelqsvtleptlsldyitceyktvipspy
vkccgtaeckdkslpdysckvftgvypfmwggaycfcdaentqlseahvekseseckteefasa
yrahtasasaklrvlyqgnnitvaayangdhavtvkdakfvvgpmssawtpfdnkivvykgd
vynmdyppfgagrpgqfgdiqsrtpeskdvyantqlvlqrpaagtvhvpysqapsgfkywlk
ergaslqhtapfgcqiatnpvravncavgnipisidipdaaftrvvdapsvtdmscevpact
hssdfggvaiikytaskkgkcavhsmtnavtireadvevegnsqlqisfstalasaefrvqv
cstqvhcaaachppkdhivnypashttlgvqdisttamswvqkitggvglivavaalilivv
lcvsfsrh

An exemplary Venezuelan equine encephalitis viral structural protein is described below (SEQ ID NO: 8): 
[Chem. 3]
mfpfqpmypmqpmpyrnpfaaprrpwfprtdpflamqvqeltrsmanltfkqrrdappegps
aakpkkeasqkqkgggqgkkkknqgkkkaktgppnpkaqngnkkktnkkpgkrqrmvmkles
dktfpimlegkingyacvvggklfrpmhvegkidndvlaalktkkaskydleyadvpqnmra
dtfkythekpqgyyswhhgavqyengrftvpkgvgakgdsgrpildnqgrvvaivlggvneg
srtalsvvmwnekgvtvkytpenceqwslvttmcllanvtfpcaqppicydrkpaetlamls
vnvdnpgydelleaavkcpgrkrrsteelfneykltrpymarcircavgschspiaieavks
dghdgyvrlqtssqygldssgnlkgrtmrydmhgtikeiplhqvslytsrpchivdghgyfl
larcpagdsitmefkkdsvrhscsvpyevkfnpvgrelythppehgveqacqvyahdaqnrg
ayvemhlpgsevdsslvslsgssvtvtppdgtsalvececggtkisetinktkqfsqctkke
qcrayrlqndkwvynsdklpkaagatlkgklhvpflladgkctvplapepmitfgfrsvslk
lhpknptylitrqladephythelisepavrnftvtekgwefvwgnhppkrfwaqetapgnp
hglphevithyyhrypmstilglsicaaiatvsvaastwlfcrsrvacltpyrltpnaripf
clavlccartaraettwesldhlwnnnqqmfwiqlliplaalivvtrllrcvccvvpflvma
gaagagayehattmpsqagisyntivnragyaplpisitptkikliptvnleyvtchyktgm
dspaikccgsqectptyrpdeqckvftgvypfmwggaycfcdtentqvskayvmksddclad
haeaykahtasvqaflnitvgehsivttvyvngetpvnfngvkitagplstawtpfdrkivq
yageiynydfpeygagqpgafgdiqsrtvsssdlyantnlvlqrpkagaihvpytqapsgfe
qwkkdkapslkftapfgceiytnpiraencavgsiplafdipdalftrvsetptlsaaectl
necvyssdfggiatvkysasksgkcavhvpsgtatlkeaavelteqgsatihfstanihpef
rlqictsyvtckgdchppkdhivthpqyhaqtftaavsktawtwltsllggsaviiiiglvl
ativamyvltnqkhn
 In one embodiment, a first attachment site comprises an amino group, preferably an amino group of a lysine residue. In one embodiment, the second attachment site comprises sulfhydryl group, preferably, a sulfhydryl group of a cysteine.

According to the present application, a Chikungunya virus-like particle or Venezuelan equine encephalitis virus-like particle comprising a Chikungunya or Venezuelan equine encephalitis viral structural protein and at least one galectin-3 antigen, wherein the at least one galectin-3 antigen is inserted in E3 of the viral structural protein, and the Chikungunya viral structural protein or Venezuelan equine encephalitis viral structural protein and the galectin-3 antigen are expressed as a fusion protein can be provided. The galectin-3 antigen may be inserted directly or indirectly in E3 of the viral structural protein.

The viral structural protein of Chikungunya virus as well as Venezuelan equine encephalitis consist of E1, E2, 6K and E3. 6K is naturally cleaved during the process of assembly and removed from the VLPs. The mature VLPs consists of capsid, E1 and E2. In the present specification and claims, "viral structural protein" refers not only those having 6K but also after 6K is removed.

6K sequences of the CHIKV and VEEV used in the working examples are as follows: CHIKV OPY-1 Strain, 6K: 749-809aa of SEQ ID NO: 6
atyqeaaiylwneqqplfwlqaliplaalivlcnclrllpcccktlaflavmsvgahtvsa (SEQ ID NO: 9) CHIKV 37997 strain, 6K: 749-809aa of SEQ ID NO: 7
atyyeaaaylwneqqplfwlqaliplaalivlcnclkllpcccktlaflavmsigahtvsa (SEQ ID NO: 10) VEEV TC-83strain, 6K: 758-813aa of SEQ ID NO: 8
ettwesldhlwnnnqqmfwiqqlliplaalivvtrllreveevvpflvmagaagaga
   (SEQ ID NO: 11)

Regarding Chikungunya viral structural protein, at least one galectin-3 antigen may be inserted instead of furin site (RKRR) from 322R to 325R of SEQ ID NO: 6 or 7. For example, regarding Chikungunya viral structural protein, at least one galectin-3 antigen is inserted between residues H at 321-position and S at 326-position of SEQ ID NO: 6 or 7; between P at 320-position and S at 326-position of SEQ ID NO: 6 or 7; or between S at 319-position and S at 326-position of SEQ ID NO: 6 or 7. VLP_CHI 0.56 vector may be used for preparing Chikungunya virus-like particle where the galectin-3 antigen is inserted between residues 321 and 326 of SEQ ID NO: 6 or 7. When an galectin-3 antigen is inserted between residues 321 and 326 of SEQ ID NO: 6 or 7, the virus-like particle provided by the present application may be Chikungunya virus-like particle consisting of a complex of E2 and E3, capsid and E1, and wherein the at least one galectin-3 antigen is inserted into E3 region.

Venezuelan equine encephalitis viral structural protein, at least one galectin-3 antigen may be inserted instead of furin site (RKRR) from 331R to 334R of SEQ ID NO: 8. For example, regarding Venezuelan equine encephalitis viral structural protein, at least one galectin-3 antigen is inserted between G at 330-position and S at 335-position of SEQ ID NO: 8; between P at 329-position and S at 335-position of SEQ ID NO: 8; or between C at 328-position and S at 335-position of SEQ ID NO: 8. VLP_VEEV 0.66 vector may be used for preparing Venezuelan equine encephalitis virus-like particle where the antigen is inserted between residues 330 and 335 of SEQ ID NO: 8. When an antigen is inserted between residues 330 and 335 of SEQ ID NO: 8, the virus-like particle provided by the present application may be Venezuelan equine encephalitis virus-like particle consisting of a complex of E2 and E3, capsid and E1, and wherein the at least one galectin-3 antigen is inserted into E3 region.

By "alteration" is meant a change in an amino acid or nucleotide at a specified position with reference to a polypeptide sequence or polynucleotide sequence. As used herein, an alteration includes a substitution, deletion, or insertion of an amino acid or nucleotide at a specified position of a polypeptide or polynucleotide. In some embodiments, an alteration in an alphavirus capsid protein nuclear localization signal includes substitution of a charged amino acid (e.g., lysine or arginine) with an uncharged amino acid (e.g., alanine or asparagine, or any amino acid except a basic charged amino acid such as lysine or arginine).

By "analog" is meant a molecule that is not identical, but has analogous functional or structural features. For example, a polypeptide analog retains the biological activity of a corresponding naturally-occurring polypeptide, while having certain biochemical modifications that enhance the analog's function relative to a naturally occurring polypeptide. Such biochemical modifications could increase the analog's protease resistance, membrane permeability, or half-life, without altering, for example, ligand binding. An analog may include an unnatural amino acid.

In this disclosure, "comprises," "comprising," "containing" and "having" and the like can have the meaning ascribed to them in U.S. Patent law and can mean " includes," "including," and the like; "consisting essentially of or "consists essentially" likewise has the meaning ascribed in U.S. Patent law and the term is open-ended, allowing for the presence of more than that which is recited so long as basic or novel characteristics of that which is recited is not changed by the presence of more than that which is recited, but excludes prior art embodiments.

"Detect" refers to identifying the presence, absence or amount of the analyte to be detected.

By "disease" is meant any condition or disorder that damages or interferes with the normal function of a cell, tissue, or organ.

By "agent" is meant any small molecule chemical compound, antibody, nucleic acid molecule, or polypeptide, or fragments thereof.

By "effective amount" is meant the amount of an agent required to ameliorate the symptoms of a disease relative to an untreated patient. The effective amount of active compound(s) used to practice the method disclosed herein for the prevention or treatment of a disease varies depending upon the manner of administration, the age, body weight, and general health of the subject. Ultimately, the attending physician or veterinarian will decide the appropriate amount and dosage regimen. Such amount is referred to as an "effective" amount.

By "ameliorate" is meant decrease, suppress, attenuate, diminish, arrest, or stabilize the development or progression of a disease or a symptom thereof.

The present disclosure provides a number of targets that are useful for the development of highly specific drugs to treat or prevent a diseases described herein. In addition, the methods disclosed herein provide a facile means to identify therapies that are safe for use in subjects. In addition, the methods provide a route for analyzing virtually any number of compounds for effects on a disease described herein with high- volume throughput, high sensitivity, and low complexity.

By "fragment" is meant a portion of a polypeptide or nucleic acid molecule. This portion contains, preferably, at least 10%, 20%, 30%, 40%, 50%, 60%, 70%, 80%, or 90% of the entire length of the reference nucleic acid molecule or polypeptide. A fragment may contain 10, 20, 30, 40, 50, 60, 70, 80, 90, or 100, 200, 300, 400, 500, 600, 700, 800, 900, or 1000 nucleotides or amino acids.

By "isolated polynucleotide" is meant a nucleic acid molecule (e.g., a DNA) that is free of the genes which, in the naturally-occurring genome of the organism from which the nucleic acid molecule is derived, flank the gene. The term therefore includes, for example, a recombinant DNA that is incorporated into a vector; into an autonomously replicating plasmid or virus; or into the genomic DNA of a prokaryote or eukaryote; or that exists as a separate molecule (for example, a cDNA or a genomic or cDNA fragment produced by PCR or restriction endonuclease digestion) independent of other sequences. In addition, the term includes an RNA molecule that is transcribed from a DNA molecule, as well as a recombinant DNA that is part of a hybrid gene encoding additional polypeptide sequence.

By an "isolated polypeptide" is meant a polypeptide that has been separated from components that naturally accompany it. Typically, the polypeptide is isolated when it is at least 60%, by weight, free from the proteins and naturally-occurring organic molecules with which it is naturally associated. Preferably, the preparation is at least 75%, more preferably at least 90%, and most preferably at least 99%, by weight, a polypeptide of the disclosure. An isolated polypeptide may be obtained, for example, by extraction from a natural source, by expression of a recombinant nucleic acid encoding such a polypeptide; or by chemically synthesizing the protein. Purity can be measured by any appropriate method, for example, column chromatography, polyacrylamide gel electrophoresis, or by HPLC analysis.

By "marker" is meant any protein or polynucleotide having an alteration in expression level or activity that is associated with a disease or disorder.

As used herein, "nuclear localization signal" or "NLS" is an amino acid sequence that, when present on the surface of a polypeptide, targets the polypeptide to the nucleus of the cell. NLS sequences are known in the art. See, for example, Non Patent Literature 19: Goldfarb, D., and N. Michaud (1991) Trends Cell Biol. 1, 20-24; Non Patent Literature 20: Gorlich, D., and I. W. Mattaj (1996) Science 271, 1513-1518). In one embodiment, an NLS includes one or more short sequences of positively charged amino acids, such as lysines or arginines. Consensus sequences for NLS include K-K/R-X-K/R (Non Patent Literature 21: Schneider, J. et al. (1988) Cell 54,117-125) and two clusters of basic amino acids, separated by a spacer of about 10 amino acids, e.g., KR[PAATKKAGQA] KKKK (Non Patent Literature 22: Dingwall et al., Cell Biol. 107 (3): 841-9). With reference to the alphavirus amino acid sequences disclosed herein, NLS are present at amino acids 67-70 of an EEEV capsid protein (KRKK); at amino acids 67-70 of an WEEV capsid protein (KKKK); at amino acids 64-68 of a VEEV capsid protein (KKPKK); at amino acids 62-69 of a CHIKV capsid protein (RRNRKNKK); at amino acids 71-74 of a Ross River virus capsid protein (RKKK); and at amino acids 64-68 of a Barmah Forest virus capsid protein (KKPKK).

As used herein, "obtaining" as in "obtaining an agent" includes synthesizing, purchasing, or otherwise acquiring the agent.

By "reduces" is meant a negative alteration of at least 10%, 25%, 50%, 75%, or 100%.

By "reference" is meant a standard or control condition.

A "reference sequence" is a defined sequence used as a basis for sequence comparison. A reference sequence may be a subset of or the entirety of a specified sequence; for example, a segment of a full-length cDNA or gene sequence, or the complete cDNA or gene sequence. For polypeptides, the length of the reference polypeptide sequence will generally be at least about 16 amino acids, preferably at least about 20 amino acids, more preferably at least about 25 amino acids, and even more preferably about 35 amino acids, about 50 amino acids, or about 100 amino acids. For nucleic acids, the length of the reference nucleic acid sequence will generally be at least about 50 nucleotides, preferably at least about 60 nucleotides, more preferably at least about 75 nucleotides, and even more preferably about 100 nucleotides or about 300 nucleotides or any integer thereabout or therebetween.

By "specifically binds" is meant a compound or antibody that recognizes and binds a polypeptide of the disclosure, but which does not substantially recognize and bind other molecules in a sample, for example, a biological sample, which naturally includes a polypeptide of the disclosure.

Nucleic acid molecules useful in the methods herein include any nucleic acid molecule that encodes a polypeptide of the disclosure or a fragment thereof. Such nucleic acid molecules need not be 100% identical with an endogenous nucleic acid sequence, but will typically exhibit substantial identity. Polynucleotides having "substantial identity" to an endogenous sequence are typically capable of hybridizing with at least one strand of a double- stranded nucleic acid molecule. Nucleic acid molecules useful in the methods of the disclosure include any nucleic acid molecule that encodes a polypeptide of the disclosure or a fragment thereof. Such nucleic acid molecules need not be 100% identical with an endogenous nucleic acid sequence, but will typically exhibit substantial identity. Polynucleotides having "substantial identity" to an endogenous sequence are typically capable of hybridizing with at least one strand of a double-stranded nucleic acid molecule. By "hybridize" is meant pair to form a double-stranded molecule between complementary polynucleotide sequences (e.g., a gene described herein), or portions thereof, under various conditions of stringency.

For example, stringent salt concentration will ordinarily be less than about 750 mM NaCl and 75 mM trisodium citrate, preferably less than about 500 mM NaCl and 50 mM trisodium citrate, and more preferably less than about 250 mM NaCl and 25 mM trisodium citrate. Low stringency hybridization can be obtained in the absence of organic solvent, e.g., formamide, while high stringency hybridization can be obtained in the presence of at least about 35% formamide, and more preferably at least about 50% formamide. Stringent temperature conditions will ordinarily include temperatures of at least about 30°C, more preferably of at least about 37°C, and most preferably of at least about 42°C. Varying additional parameters, such as hybridization time, the concentration of detergent, e.g., sodium dodecyl sulfate (SDS), and the inclusion or exclusion of carrier DNA, are well known to those skilled in the art. Various levels of stringency are accomplished by combining these various conditions as needed. In a preferred: embodiment, hybridization will occur at 30°C in 750 mM NaCl, 75 mM trisodium citrate, and 1% SDS. In a more preferred embodiment, hybridization will occur at 37°C in 500 mM NaCl, 50 mM trisodium citrate, 1% SDS, 35% formamide, and 100 µg/ml denatured salmon sperm DNA (ssDNA). In a most preferred embodiment, hybridization will occur at 42°C in 250 mM NaCl, 25 mM trisodium citrate, 1% SDS, 50% formamide, and 200 µg/ml ssDNA. Useful variations on these conditions will be readily apparent to those skilled in the art.

For most applications, washing steps that follow hybridization will also vary in stringency. Wash stringency conditions can be defined by salt concentration and by temperature. As above, wash stringency can be increased by decreasing salt concentration or by increasing temperature. For example, stringent salt concentration for the wash steps will preferably be less than about 30 mM NaCl and 3 mM trisodium citrate, and most preferably less than about 15 mM NaCl and 1.5 mM trisodium citrate. Stringent temperature conditions for the wash steps will ordinarily include a temperature of at least about 25 °C, more preferably of at least about 42°C, and even more preferably of at least about 68°C. In a preferred embodiment, wash steps will occur at 25 °C in 30 mM NaCl, 3 mM trisodium citrate, and 0.1% SDS. In a more preferred embodiment, wash steps will occur at 42 C in 15 mM NaCl, 1.5 mM trisodium citrate, and 0.1% SDS. In a more preferred embodiment, wash steps will occur at 68°C in 15 mM NaCl, 1.5 mM trisodium citrate, and 0.1% SDS.

Additional variations on these conditions will be readily apparent to those skilled in the art. Hybridization techniques are well known to those skilled in the art and are described, for example, in Benton and Davis (Science 196:180, 1977); Grunstein and Hogness (Proc. Natl. Acad. Sci., USA 72:3961, 1975); Ausubel et al. (Current Protocols in Molecular Biology, Wiley Interscience, New York, 2001); Berger and Kimmel (Guide to Molecular Cloning Techniques, 1987, Academic Press, New York); and Sambrook et al., Molecular Cloning: A Laboratory Manual, Cold Spring Harbor Laboratory Press, New York. By "substantially identical" is meant a polypeptide or nucleic acid molecule exhibiting at least 50% identity to a reference amino acid sequence (for example, any one of the amino acid sequences described herein) or nucleic acid sequence (for example, any one of the nucleic acid sequences described herein). Preferably, such a sequence is at least 60%, more preferably 80% or 85%, and more preferably 90%, 95% or even 99% identical at the amino acid level or nucleic acid to the sequence used for comparison.

Sequence identity is typically measured using sequence analysis software (for example, Sequence Analysis Software Package of the Genetics Computer Group, University of Wisconsin Biotechnology Center, 1710 University Avenue, Madison, Wis. 53705, BLAST, BESTFIT, GAP, or PILEUP/PRETTYBOX programs). Such software matches identical or similar sequences by assigning degrees of homology to various substitutions, deletions, and/or other modifications. Conservative substitutions typically include substitutions within the following groups: glycine, alanine; valine, isoleucine, leucine; aspartic acid, glutamic acid, asparagine, glutamine; serine, threonine; lysine, arginine; and phenylalanine, tyrosine. In an exemplary approach to determining the degree of identity, a BLAST program may be used, with a probability score between e<"3> and e<"100> indicating a closely related sequence.

By "subject" is meant a mammal, including, but not limited to, a human or non- human mammal, such as a bovine, equine, canine, ovine, or feline.

Ranges provided herein are understood to be shorthand for all of the values within the range. For example, a range of 1 to 50 is understood to include any number, combination of numbers, or sub-range from the group consisting 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50.

As used herein, the terms "treat," treating," "treatment," and the like refer to reducing or ameliorating a disorder and/or symptoms associated therewith. It will be appreciated that, although not precluded, treating a disorder or condition does not require that the disorder, condition or symptoms associated therewith be completely eliminated.

Unless specifically stated or obvious from context, as used herein, the term "or" is understood to be inclusive.

Unless specifically stated or obvious from context, as used herein, the terms "a", "an", and "the" are understood to be singular or plural.

Unless specifically stated or obvious from context, as used herein, the term "about" is understood as within a range of normal tolerance in the art, for example within 2 standard deviations of the mean. About can be understood as within 10%, 9%, 8%, 7%, 6%, 5%, 4%, 3%, 2%, 1%, 0.5%, 0.1%, 0.05%, or 0.01% of the stated value. Unless otherwise clear from context, all numerical values provided herein are modified by the term about.

The recitation of a listing of chemical groups in any definition of a variable herein includes definitions of that variable as any single group or combination of listed groups. The recitation of an embodiment for a variable or aspect herein includes that embodiment as any single embodiment or in combination with any other embodiments or portions thereof.

Any compositions or methods provided herein can be combined with one or more of any of the other compositions and methods provided herein.

### Alphavirus Polynucleotides

In general, the disclosure includes any nucleic acid sequence encoding a VLP having an alteration in a structural protein that enhances VLP expression in a mammalian cell. In one embodiment, the alphavirus polypeptide(s) includes at least an alphavirus E2 protein or capsid protein NLS comprising an alteration that increases VLP expression in a mammalian cell. In one embodiment, the alphavirus E2 protein has a non-lysine residue (e.g., asparagine) at the amino acid position corresponding to amino acid 234 in the CHIKV E2 protein and/or a modification at the amino acid position corresponding to amino acid 251 in the CHIKV E2 protein that destabilizes the E2 protein during viral budding. In another embodiment, the alphavirus polypeptide(s) includes at least an alphavirus capsid protein having a non-lysine residue (e.g., alanine or asparagine) at an amino acid position corresponding to a lysine residue in an alphavirus capsid protein NLS and/or a non-arginine residue (e.g., alanine or asparagine) at an amino acid position corresponding to a arginine residue in an alphavirus capsid protein NLS. In specific embodiments, the alphavirus capsid protein is a WEEV CBA87 strain capsid protein having one or more of the alterations K67N, K68N, and/or K69N. In certain embodiments, the alphavirus capsid protein is a VEEV TC83 strain capsid protein having one or more of the alterations K64N, K65A, K65N, K67A, and/or K67N. In some embodiments, the alphavirus capsid protein is a EEEV PE-6 strain capsid protein having an alteration K67N. In particular embodiments, the alphavirus capsid protein is a CHIKV(Strain 37997) strain capsid protein having one or more of the alterations R62A, R63A, R65A, K66A, K68A, and/or K69A. In specific embodiments, the alphavirus capsid protein is a Ross River Virus capsid protein having one or more of the alterations R71N, K72N, K73N, and/or K74N. In specific embodiments, the alphavirus capsid protein is a Barmah Forest Virus capsid protein having one or more of the alterations K64A, K64N, K65A, K65N, K67A, K67N, K68A and/or K68N. An isolated nucleic acid molecule can be manipulated by recombinant DNA techniques well known in the art. Thus, a nucleotide sequence contained in a vector in which 5' and 3' restriction sites are known, or for which polymerase chain reaction (PCR) primer sequences have been disclosed, is considered isolated, but a nucleic acid sequence existing in its native state in its natural host is not. In certain exemplary embodiments, the vector comprises CHIKV 37997 or WEEV 71V-1658 nucleic acid segments, or fragments thereof. The vector may further comprise a CMV/R promoter. The vector may also comprise the capsid protein, or a fragment thereof.

In other exemplary embodiments, in addition to the E2 protein, the vector comprises another envelope protein selected from the group consisting of E3, 6K, and El. In certain examples, the vaccine may comprise capsid, E3, E2, 6K and El. In other examples, the vaccine may comprise E3, E2, 6K and El.

In a particular embodiment, a nucleic acid molecule set forth in the sequences disclosed herein includes a nucleotide sequence encoding a polypeptide having at least about 50%, 60%, 70%, 75%, 80%, 81%, 82%, 83%, 84%, 85%, 86%, 87%, 88%, 89%, 90%, 91%, 92%, 93%, 94%, 95%, 96%, 97%, 98%, or more identity (e.g., when compared to the overall length of the amino acid sequence) to a polypeptide encoding a protein selected from alphavirus capsid, E3, E2, 6K and El, including CHIKV or VEEV capsid, E3, E2, 6K and El.

In some embodiments of the disclosure, proteins may comprise mutations containing alterations which produce silent substitutions, additions, or deletions, but do not alter the properties or activities of the encoded protein or how the proteins are made. Nucleotide variants can be produced for a variety of reasons, e.g., to optimize codon expression for a particular host, see U.S. patent publication 2005/0118191, herein incorporated by reference in its entirety for all purposes.

In addition, the nucleotides can be sequenced to ensure that the correct coding regions were cloned and do not contain any unwanted mutations. The nucleotides can be subcloned into an expression vector (e.g., baculovirus) for expression in any cell. A person with skill in the art understands that various subcloning methods are available and are possible.

An isolated nucleic acid may be substantially purified, but need not be. For example, a nucleic acid that is isolated within a cloning or expression vector is not pure in that it may comprise only a tiny percentage of the material in the cell in which it resides. Such a nucleic acid is isolated, as the term is used herein, because it is readily manipulatable by standard techniques known to those of ordinary skill in the art.

### Polypeptide Expression

In general, VLPs comprising one or more alphavirus polypeptides herein may be produced by transformation of a suitable host cell with all or part of a polypeptide-encoding nucleic acid molecule or fragment thereof in a suitable expression vehicle.

Those skilled in the field of molecular biology will understand that any of a wide variety of expression systems may be used to provide the recombinant protein. The precise host cell used is not critical to the invention. A polypeptide of the disclosure may be produced in a prokaryotic host (e.g., E. coli) or in a eukaryotic host (e.g., Saccharomyces cerevisiae, insect cells, e.g., Sf21 cells, or mammalian cells, e.g., NIH 3T3, HeLa, COS cells). Such cells are available from a wide range of sources (e.g., the American Type Culture Collection, Rockland, Md.; also, see, e.g., Ausubel et al., supra). Non limiting examples of insect cells are, Spodoptera frugiperda (Sf) cells, e.g., Sf9, Sf21, Trichoplusia ni cells, e.g., High Five cells, and Drosophila S2 cells. Examples of fungi (including yeast) host cells are S. cerevisiae, Kluyveromyces lactis (K lactis), species of Candida including C. albicans and C. glabrata, Aspergillus nidulans, Schizosaccharomyces pombe (S. pombe), Pichia pastoris, and Yarrowia lipolytica. Examples of mammalian cells are COS cells, baby hamster kidney cells, mouse L cells, LNCaP cells, Chinese hamster ovary (CHO) cells, human embryonic kidney (HEK) cells, African green monkey cells, CV1 cells, HeLa cells, MDCK cells, Vero and Hep-2 cells. Xenopus laevis oocytes, or other cells of amphibian origin, may also be used. Prokaryotic host cells include bacterial cells, for example, E. coli, B. subtilis, and mycobacteria.

Methods of cloning said proteins are known in the art. For example, the gene encoding a specific alphavirus protein, e.g., a CHIKV, WEEV, EEEV, VEEV, Ross River virus, or Barmah Forest virus structural protein can be isolated by RT-PCR from polyadenylated mRNA extracted from cells which had been infected with said virus. The resulting product gene can be cloned as a DNA insert into a vector. The term "vector" refers to the means by which a nucleic acid can be propagated and/or transferred between organisms, cells, or cellular components. Vectors include plasmids, viruses, bacteriophages, pro-viruses, phagemids, transposons, artificial chromosomes, and the like, that replicate autonomously or can integrate into a chromosome of a host cell. A vector can also be a naked RNA polynucleotide, a naked DNA polynucleotide, a polynucleotide composed of both DNA and RNA within the same strand, a poly-lysine-conjugated DNA or RNA, a peptide-conjugated DNA or RNA, a liposome-conjugated DNA, or the like, that is not autonomously replicating. In many, but not all, common embodiments, the vectors of the present invention are plasmids or bacmids.

The invention further provides nucleotides that encode proteins, including chimeric molecules, cloned into an expression vector that can be expressed in a cell that provides for the formation of VLPs. An "expression vector" is a vector, such as a plasmid, that is capable of promoting expression, as well as replication of a nucleic acid incorporated therein.

Typically, the nucleic acid molecule to be expressed is "operably linked" to a promoter and/or enhancer, and is subject to transcription regulatory control by the promoter and/or enhancer.

A variety of expression systems exist for the production of the polypeptides of the disclosure. Expression vectors useful for producing such polypeptides include, without limitation, chromosomal, episomal, and virus-derived vectors, e.g., vectors derived from bacterial plasmids, from bacteriophage, from transposons, from yeast episomes, from insertion elements, from yeast chromosomal elements, from viruses such as baculoviruses, papova viruses, such as SV40, vaccinia viruses, adenoviruses, fowl pox viruses, pseudorabies viruses and retroviruses, and vectors derived from combinations thereof.

Constructs and/or vectors provided herein comprise alphavirus polynucleotides that encode structural polypeptides, including envelope proteins or capsid proteins or portions thereof as described herein. The vector may be, for example, a phage, plasmid, viral, or retroviral vector. The constructs and/or vectors that comprise the nucleotides should be operatively linked to an appropriate promoter, such as the CMV promoter, phage lambda PL promoter, the E. coli lac, phoA and tac promoters, the SV40 early and late promoters, and promoters of retroviral LTRs are non-limiting examples. Other suitable promoters will be known to the skilled artisan depending on the host cell and/or the rate of expression desired. The expression constructs will further contain sites for transcription initiation, termination, and, in the transcribed region, a ribosome-binding site for translation. The coding portion of the transcripts expressed by the constructs will preferably include a translation initiating codon at the beginning and a termination codon appropriately positioned at the end of the polypeptide to be translated.

Expression vectors will preferably include at least one selectable marker. Such markers include dihydrofolate reductase, G418 or neomycin resistance for eukaryotic cell culture and tetracycline, kanamycin or ampicillin resistance genes for culturing in E. coli and other bacteria. Among vectors preferred are virus vectors, such as baculovirus, poxvirus (e.g., vaccinia virus, avipox virus, canarypox virus, fowlpox virus, raccoonpox virus, swinepox virus, etc.), adenovirus (e.g., canine adenovirus), herpesvirus, and retrovirus. Other vectors that can be used with the invention comprise vectors for use in bacteria, which comprise pQE70, pQE60 and pQE-9, pBluescript vectors, Phagescript vectors, pNH8A, pNH16a, pNH18A, pNH46A, ptrc99a, pKK223-3, pKK233-3, pDR540, pRIT5. Among preferred eukaryotic vectors are pFastBacl pWINEO, pSV2CAT, pOG44, pXTl and pSG, pSVK3, pBPV, pMSG, and pSVL. Other suitable vectors will be readily apparent to the skilled artisan.

Recombinant constructs can be prepared and used to transfect, infect, or transform and can express viral proteins, including those described herein, into eukaryotic cells and/or prokaryotic cells. Thus, the invention provides for host cells which comprise a vector (or vectors) that contain nucleic acids which code for alphavirus structural genes, including capsid, E3, E2, 6K, and El or portions thereof, and/or any chimeric molecule described above, and permit the expression of alphavirus structural genes, including capsid E3, E2, 6K, and El, or portions thereof, and/or any chimeric molecule described above in said host cell under conditions which allow the formation of VLPs.

In one embodiment, said vector is a recombinant baculovirus. In another embodiment, said recombinant baculovirus is transfected into an insect cell. In a preferred embodiment, said cell is an insect cell. In another embodiment, said insect cell is a Sf9 cell.

In another embodiment, said vector and/or host cell comprises nucleotides that encode alphavirus genes, including capsid, E3, E2, 6K, and El, or portions thereof as described
In another embodiment, said vector and/or host cell consists essentially of alphavirus capsid, E3, E2, 6K, and El, or portions thereof as described herein. In a further embodiment, said vector and/or host cell consists of alphavirus protein comprising capsid, E3, E2, 6K, and El, or portions thereof, as described herein. These vector and/or host cell contain alphavirus core, E3, E2, 6K, and El, or portions thereof, as described herein, and may contain additional cellular constituents such as cellulVLProteins, baculovirus proteins, lipids, carbohydrates etc.

One particular bacterial expression system for polypeptide production is the E. coli pET expression system (Novagen, Inc., Madison, Wis). According to this expression system, DNA encoding a polypeptide is inserted into a pET vector in an orientation designed to allow expression. Since the gene encoding such a polypeptide is under the control of the T7 regulatory signals, expression of the polypeptide is achieved by inducing the expression of T7 RNA polymerase in the host cell. This is typically achieved using host strains that express T7 RNA polymerase in response to IPTG induction. Once produced, a recombinant polypeptide is then isolated according to standard methods known in the art, for example, those described herein.

Another bacterial expression system for polypeptide production is the pGEX expression system (Pharmacia). This system employs a GST gene fusion system that is designed for high-level expression of genes or gene fragments as fusion proteins with rapid purification and recovery of functional gene products. The protein of interest is fused to the carboxyl terminus of the glutathione S-transferase protein from Schistosoma japonicum and is readily purified from bacterial lysates by affinity chromatography using Glutathione Sepharose 4B. Fusion proteins can be recovered under mild conditions by elution with glutathione. Cleavage of the glutathione S-transferase domain from the fusion protein is facilitated by the presence of recognition sites for site-specific proteases upstream of this domain. For example, proteins expressed in pGEX-2T plasmids may be cleaved with thrombin; those expressed in pGEX-3X may be cleaved with factor Xa.

### Alphavirus Polypeptides and Analogs

The invention provides VLPs comprising one or more alphavirus polypeptides. Also included in the invention are VLPs comprising one or more alphavirus polypeptides or fragments thereof that are modified in ways that enhance or do not inhibit their ability to modulate an immune response. In one embodiment, the invention provides methods for optimizing an alphavirus amino acid sequence or nucleic acid sequence by producing an alteration. Such alterations may include certain mutations, deletions, insertions, or post-translational modifications. The invention further includes analogs of any naturally-occurring polypeptide of the disclosure. Analogs can differ from the naturally-occurring the polypeptide of the disclosure by amino acid sequence differences, by post-translational modifications, or by both. Analogs of the disclosure will generally exhibit at least 85%, more preferably 90%, and most preferably 95% or even 99% identity with all or part of a naturally-occurring amino, acid sequence of the disclosure. The length of sequence comparison is at least 10, 13, 15 amino acid residues, preferably at least 25 amino acid residues, and more preferably more than 35 amino acid residues.

Alterations of an alphavirus polypeptide include but are not limited to site-directed, random point mutagenesis, homologous recombination (DNA shuffling), mutagenesis using uracil containing templates, oligonucleotide-directed mutagenesis, phosphorothioate- modified DNA mutagenesis, mutagenesis using gapped duplex DNA or the like. Additional suitable methods include point mismatch repair, mutagenesis using repair-deficient host strains, restriction-selection and restriction-purification, deletion mutagenesis, mutagenesis by total gene synthesis, double-strand break repair, and the like. Mutagenesis, e.g., involving chimeric constructs, is also included in the present invention. In one embodiment, mutagenesis can be guided by known information of the naturally occurring molecule or altered or mutated naturally occurring molecule, e.g., sequence, sequence comparisons, physical properties, crystal structure or the like.

In one embodiment, the invention provides polypeptide variants that differ from a reference polypeptide. The term "variant" refers to an amino acid sequence that is altered by one or more amino acids with respect to a reference sequence. The variant can have "conservative" changes, wherein a substituted amino acid has similar structural or chemical properties, e.g., replacement of leucine with isoleucine. Alternatively, a variant can have "nonconservative" changes, e.g., replacement of a glycine with a tryptophan. Analogous minor variations can also include amino acid deletion or insertion, or both. Guidance in determining which amino acid residues can be substituted, inserted, or deleted without eliminating biological or immunological activity can be found using computer programs well known in the art, for example, DNASTAR software. Desirably, variants show substantial biological activity. In one embodiment, a protein variant forms an VLP and elicits an antibody response when administered to a subject.

Natural variants can occur due to mutations in the proteins. These mutations may lead to antigenic variability within individual groups of infectious agents. Thus, a person infected with a particular strain develops antibody against that virus, as newer virus strains appear, the antibodies against the older strains no longer recognize the newer virus and reinfection can occur. The invention encompasses all antigenic and genetic variability of proteins from infectious agents for making VLPs.

In addition to full-length polypeptides, the invention also includes fragments of any one of the polypeptides of the disclosure. As used herein, the term "a fragment" means at least 5, 10, 13, or 15. In other embodiments a fragment is at least 20 contiguous amino acids, at least 30 contiguous amino acids, or at least 50 contiguous amino acids, and in other embodiments at least 60 to 80 or more contiguous amino acids. Fragments of the disclosure can be generated by methods known to those skilled in the art or may result from normal protein processing (e.g., removal of amino acids from the nascent polypeptide that are not required for biological activity or removal of amino acids by alternative mRNA splicing or alternative protein processing events).

Non-protein analogs having a chemical structure designed to mimic alphavirus VLPs or one or more alphavirus polypeptides functional activity can be administered according to methods of the disclosure. Alphavirus analogs may exceed the physiological activity of native alphavirus.

Methods of analog design are well known in the art, and synthesis of analogs can be carried out according to such methods by modifying the chemical structures such that the resultant analogs exhibit the immunomodulatory activity of a native alphavirus polypeptide. These chemical modifications include, but are not limited to, substituting alternative R groups and varying the degree of saturation at specific carbon atoms of the native alphavirus molecule.

Preferably, the analogs are relatively resistant to in vivo degradation, resulting in a more prolonged therapeutic effect upon administration. Assays for measuring functional activity include, but are not limited to, those described in the Examples below.

### Pharmaceutical Compositions and Administration

The invention features pharmaceutical compositions that comprise VLPs comprising the alphavirus viral structural protein and a galectin-3 antigen as described herein. The pharmaceutical compositions useful herein contain a pharmaceutically acceptable carrier, including any suitable diluent or excipient, which includes any pharmaceutical agent that does not itself induce the production of an immune response harmful to the vertebrate receiving the composition, and which may be administered without undue toxicity and a VLP of the disclosure. As used herein, the term "pharmaceutically acceptable" means being approved by a regulatory agency of the Federal or a state government or listed in the U.S. Pharmacopia, European Pharmacopia or other generally recognized pharmacopia for use in mammals, and more particularly in humans. These compositions can be useful as a vaccine and/or antigenic compositions for inducing a protective immune response in a vertebrate.

Pharmaceutically acceptable carriers include but are not limited to saline, buffered saline, dextrose, water, glycerol, sterile isotonic aqueous buffer, and combinations thereof. A thorough discussion of pharmaceutically acceptable carriers, diluents, and other excipients is presented in Remington's Pharmaceutical Sciences (Mack Pub. Co. N.J. current edition). The formulation should suit the mode of administration. In a preferred embodiment, the formulation is suitable for administration to humans, preferably is sterile, non-particulate and/or non-pyrogenic.

The composition, if desired, can also contain minor amounts of wetting or emulsifying agents, or pH buffering agents. The composition can be a solid form, such as a lyophilized powder suitable for reconstitution, a liquid solution, suspension, emulsion, tablet, pill, capsule, sustained release formulation, or powder. Oral formulation can include standard carriers such as pharmaceutical grades of mannitol, lactose, starch, magnesium stearate, sodium saccharine, cellulose, magnesium carbonate, etc.

In certain embodiments, the VLP composition is supplied in liquid form, for example in a sealed container indicating the quantity and concentration of the VLP composition.

Preferably, the liquid form of the VLP composition is supplied in a hermetically sealed container at least about 50 µg/ml, more preferably at least about 100 µg/ml, at least about 200 µg/ml, at least 500 µg/ml, or at least 1 mg/ml.

Alternatively, the vaccine formulation is administered intranasally, either by drops, large particle aerosol (greater than about 10 microns), or spray into the upper respiratory tract or small particle aerosol (less than 10 microns) or spray into the lower respiratory tract. While any of the above routes of delivery results in an immune response, intranasal administration confers the added benefit of eliciting mucosal immunity at the site of entry of many viruses, including alphaviruses, for example CHIKV or VEEV.

Thus, the invention also comprises a method of formulating a vaccine or antigenic composition that induces immunity to an infection or at least one symptom thereof to a mammal, comprising adding to said formulation an effective dose of VLPs, e.g., alphavirus (e.g., CHIKV or VEEV).

In certain cases, stimulation of immunity with a single dose is preferred, however additional dosages can be also be administered, by the same or different route, to achieve the desired effect. In neonates and infants, for example, multiple administrations may be required to elicit sufficient levels of immunity. Administration can continue at intervals throughout childhood, as necessary to maintain sufficient levels or protection.

Similarly, adults who are particularly susceptible to repeated or serious infections, such as, for example, health care workers, day care workers, family members of young children, the elderly, and individuals with compromised cardiopulmonary function or immune systems may require multiple immunizations to establish and/or maintain protective immune responses. Levels of induced immunity can be monitored, for example, by measuring amounts of neutralizing secretory and serum antibodies, and dosages adjusted or vaccinations repeated as necessary to elicit and maintain desired levels of protection.

The dosage of the pharmaceutical formulation can be determined readily by the skilled artisan, for example, by first identifying doses effective to elicit a prophylactic or therapeutic immune response, e.g., by measuring the serum titer of virus specific immunoglobulins or by measuring the inhibitory ratio of antibodies in serum samples, or urine samples, or mucosal secretions. Said dosages can be determined from animal studies. A non-limiting list of animals used to study the efficacy of vaccines include the guinea pig, hamster, ferrets, chinchilla, mouse and cotton rat, and non-human primates. Most animals are not natural hosts to infectious agents but can still serve in studies of various aspects of the disease. For example, any of the above animals can be dosed with a vaccine candidate, e.g., VLPs of the disclosure, to partially characterize the immune response induced, and/or to determine if any neutralizing antibodies have been produced. For example, many studies have been conducted in the mouse model because mice are small size and their low cost allows researchers to conduct studies on a larger scale.

In addition, human clinical studies can be performed to determine the preferred effective dose for humans by a skilled artisan. Such clinical studies are routine and well known in the art. The precise dose to be employed will also depend on the route of administration. Effective doses may be extrapolated from dose-response curves derived from in vitro or animal test systems.

As also well known in the art, the immunogenicity of a particular composition can be enhanced by the use of non-specific stimulators of the immune response, known as adjuvants. As used herein, the term "adjuvant" is meant to refer to a compound that, when used in combination with a specific immunogen in a formulation, will augment, alter or modify the resultant immune response. Adjuvants have been used experimentally to promote a generalized increase in immunity against unknown antigens. Immunization protocols have used adjuvants to stimulate responses for many years, and as such, adjuvants are well known to one of ordinary skill in the art. Some adjuvants affect the way in which antigens are presented. For example, the immune response is increased when protein antigens are precipitated by alum. Emulsification of antigens also prolongs the duration of antigen presentation. The inclusion of any adjuvant described in Vogel et al., "A Compendium of Vaccine Adjuvants and Excipients (2nd Edition)," herein incorporated by reference in its entirety for all purposes, is envisioned within the scope of this invention.

Exemplary adjuvants include complete Freund's adjuvant (a non-specific stimulator of the immune response containing killed Mycobacterium tuberculosis), incomplete Freund's adjuvants and aluminum hydroxide adjuvant. Other adjuvants comprise GMCSP, BCG, aluminum hydroxide, MDP compounds, such as thur-MDP and nor-MDP, CGP (MTP-PE), lipid A, and monophosphoryl lipid A (MPL). RIBI, which contains three components extracted from bacteria, MPL, trehalose dimycolate (TDM) and cell wall skeleton (CWS) in a 2% squalene/Tween-80 emulsion also is contemplated. MF-59, Novasomes.RTM., MHC antigens may also be used.

The VLPs of the disclosure can also be formulated with "immune stimulators." These are the body's own chemical messengers (cytokines) to increase the immune system's response. Immune stimulators include, but not limited to, various cytokines, lymphokines and chemokines with immunostimulatory, immunopotentiating, and pro-inflammatory activities, such as interleukins (e.g., IL-1, IL-2, IL-3, IL-4, IL-12, IL-13); growth factors (e.g., granulocyte-macrophage (GM)-colony stimulating factor (CSF)); and other immunostimulatory molecules, such as macrophage inflammatory factor, Flt3 ligand, B7.1; B7.2, etc. The immunostimulatory molecules can be administered in the same formulation as the VLPs, or can be administered separately. Either the protein or an expression vector encoding the protein can be administered to produce an immunostimulatory effect. Thus in one embodiment, the invention comprises antigenic and vaccine formulations comprising an adjuvant and/or an immune stimulator.

Obesity increases the risk of various diseases like hyperlipidemia, ischemic heart disease, cerebrovascular disease, diabetes, dementia and so on. Accordingly, the VLPs of the disclosure can also be used to treat, prevent or reduce the risk of those diseases.

The present application will be described in detail with reference to the following examples, which, however, are not intended to limit the scope of the present application.

### Example

### (1) Preparation of VEEV VLPs having a Gal-3 antigen (VEEV-Gal-3 VLP)

Galectin-3 (Gal-3) vaccine epitope was designed by using protein amino acid sequences and 3D-structure of human and mouse Gal-3 proteins (Table 1). Linkers SGG (N-terminal) and GGS (C-terminal) were attached to the epitope peptide SEQ ID NO: 5. For the generation of a mammalian expression vector, pVEEV Gal-3 plasmid DNA vector, a synthesized dsDNA fragment of Gal-3 epitope peptide sequence (gBlocks, IDT) was inserted into specific location (between G at 330-position and S at 335-position) of DNA encoding VEEV E3 envelope protein in pVEEV vector to obtain VEEV-Gal-3 VLP according to the procedures disclosed in WO 2022/225057.

**[Table 1]**

| **Gal-3 Epitope Peptide** | | |
|---|---|---|
| Epitope Peptide SEQ ID NO: 12 | Length (A.A.) | DNA Sequence SEQ ID NO: 13 |
| SGGADSFSLN DALAGSGNPN PQGYPGAWGN QPAGGS | 36 | GGC GCG GAC TCT TTT TCC TTG AAT GAT GCT CTC GCT GGG TCC GGT AAT CCT AAC CCA CAA GGC TAT CCT GGAGCG TGG GGC AAT CAA CCG GCG GGC |

FreeStyle 293F cells (Thermo Fisher Scientific, #R790-07) were cultured in suspension with FreeStyle 293 Expression medium (Thermo Fisher Scientific, #12338-018) using shaking incubator with 8% CO₂ at 37°C. 293F cells were transfected with pVEEV Gal-3 VLP plasmid DNA expression vector to the cells by PEI (Polyethylenimine, Polysciences, #23966) at the conditions of 0.8 µg DNA/10⁶ cells, DNA: PEI =1:3 (w/w), and 1.25 x10⁶ cells/mL culture medium. The culture supernatant was harvested 4 days post-transfection and clarified by centrifugation (3,000rpm, 10 min) followed by filtration with a 0.45 µm PES membrane filter system (VWR #10040-470).

The VLP-containing culture supernatant was layered onto 1.5 mL OptiPrep (60 % w/v) *Iodixanol,* Accurate chemical, #AN1114542) and centrifuged at 52,000 x g for 1.5 hr in SW-28 rotor (Beckmann). After removing supernatant to leave 1.5 mL above the interface, two layers were mixed to make 50 % OptiPrep solution and centrifuged at 360,000 x g for 2.5 hrs in NVT100 rotor (Beckman) to form a density gradient.

Crude VLPs were collected and purified by size exclusion chromatography using Biologic Duo-Flow FPLC system (Bio-Rad) with Hiprep 16/60 Sephacryl S-500 HR column (GE, #28-9356-06) and phosphate-buffered saline (PBS). Fractions containing VLPs were concentrated by Amicon Ultra-15 centrifugal filter units (EMD Millipore, #UFC910024) and filtered through a 0.20 µm PES membrane.

Total protein concentration was measured by BCA Protein Assay (Pierce, #23225) following the manufacturer's instructions. Purity of the VLPs was confirmed by SDS-PAGE analysis (Any kD Mini-PROTEAN TGX Precast Protein Gel, Bio-Rad, #456-9035) followed by Coomassie dye-based staining using QC Colloidal Coomassie Stain (Bio-Rad, #1610803). Sucrose and EDTA were added to the VLP solution at the final concentration of 250 mM and 5 mM, respectively. Protein concentration of VLP samples were adjusted to 0.4 mg/mL and store at -80°C.

Molecular size and purity of VEEV VLPs comprising Gal-3 epitope peptide was analyzed by SDS-PAGE under denatured condition. The obtained VLP was matched with the theoretical molecular weight and the purity was more than 90%. Protein bands regarding the fusion protein Gal-3 peptide with VEEV viral structural protein was confirmed. The obtained VLP was referred as "VEEV-Gal-3 VLP" in the following study.

### (2) Detection of anti-mouse galectin-3 (Gal-3) antibody in the serum of VEEV-Gal-3-VLP immunized mice

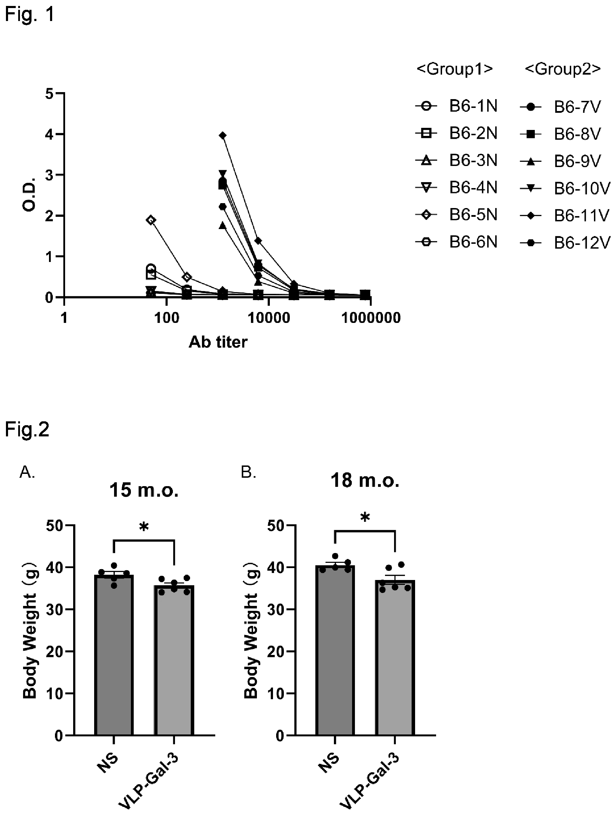

Twelve (12) months old C57BL/6J mice were used as an animal model having increased visceral fat. Twelve (12) months old mice were divided into two groups. The VEEV-Gal-3 VLP was intramuscularly administered to one group of mice (Group 2) and normal saline to the other group (Group 1). The mice were administered with the VLP or saline at the beginning of the experiment, two weeks, four weeks and three months after the first administration. Six(6) weeks after the beginning of the experiment, blood samples were obtained from each mouse and serum was prepared. Anti-mouse Gal-3 antibody in the serum was detected using ELISA where mouse Gal-3 protein was coated on ELISA plate. The results show that VEEV-Gal-3 VLP induced anti-mouse Gal-3 antibodies in Group 2 mouse (see Fig.1).

### (3) Effect of VEEV-Gal-3 VLP on body weight and visceral fat in C57BL/6J mice.

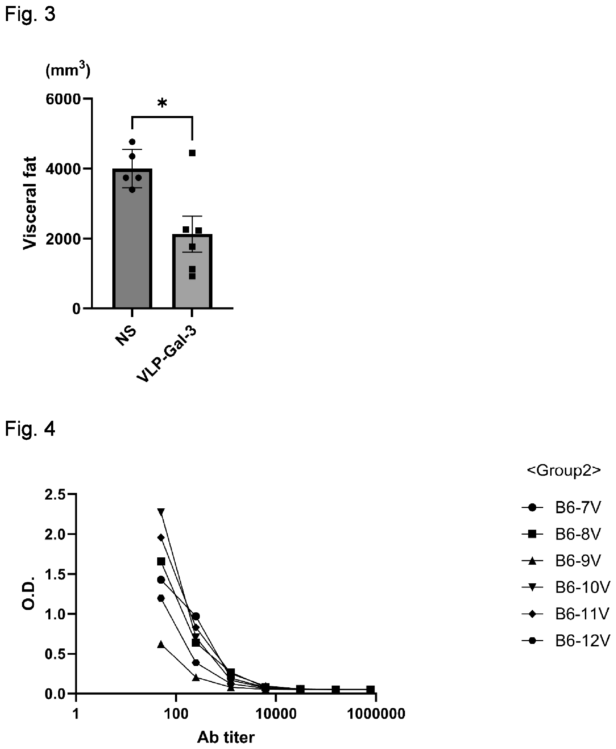

Three(3) and six(6) months after the beginning of the experiment, body weight of the animal was measured. The result showed VLP-Gal-3 injected group decreased their body weight (Fig.2). Then, mice were sacrificed and heart, part of liver and kidneys were excised. After the skin incision closure, visceral fat was measured by magnetic resonance imaging (MRI). The results showed visceral fat decreased in VEEV-Gal-3-VLP injected group (Fig.3)

### (4) Detection of anti-human galectin-3 (Gal-3) antibody in the serum of VEEV-Gal-3-VLP immunized mice

Six(6) weeks after the beginning of the experiment, blood samples were obtained from each of the VEEV-Gal-3 VLP-immunized mice (Group 2) and serum was prepared. Anti-human Gal-3 antibody in the serum was detected using ELISA where human Gal-3 protein was coated on ELISA plate. The results show that VEEV-Gal-3 VLP induced not only anti-mouse Gal-3 antibodies but also anti-human Gal-3 antibodies.

## Claims

1. A pharmaceutical composition for use in the treatment or prevention of obesity in a mammalian subject in need thereof, which comprises an effective amount of a virus-like particle comprising a viral structural protein and a galectin-3 antigen.

2. The pharmaceutical composition according to claim 1, wherein the viral structural protein is derived from alphavirus or flavivirus.

3. The pharmaceutical composition according to claim 2, wherein the viral structural protein is derived from Chikungunya virus or Venezuelan equine encephalitis virus.

4. The pharmaceutical composition according to claim 3, wherein the viral structural protein is derived from Chikungunya virus strain 37997 or strain OPY-1, or Venezuelan equine encephalitis virus strain TC-83.

5. The pharmaceutical composition according to any one of claims 1-4, wherein at least one galectin-3 antigen is inserted into the envelope protein E3 of the viral structural protein.

6. The pharmaceutical composition according to any one of claims 1-5, wherein the galectin-3 antigen is selected from the peptides of SEQ ID NO: 2, SEQ ID NO: 3, SEQ ID NO: 4 and SEQ ID NO: 5.

7. The pharmaceutical composition according to any one of claims 1-5, wherein the galectin-3 antigen is a peptide of SEQ ID NO: 12.

8. The pharmaceutical composition according to any one of claims 5-7, wherein the at least one galectin-3 antigen is inserted between residues corresponding to 321 and 326 of SEQ ID NO: 1, residues 321 and 326 of SEQ ID NO: 2 or residues 330 and 335 of SEQ ID NO: 3.

9. A pharmaceutical composition or vaccine composition for the treatment or prevention of obesity in a mammalian subject, which comprises an effective amount of a virus-like particle comprising a viral structural protein and a galectin-3 antigen.

10. A method for the treatment or prevention of obesity in a mammalian subject in need thereof, which comprises administering an effective amount of a virus-like particle comprising a viral structural protein and a galectin-3 antigen to the subject.
